# EUROPEAN PATENT APPLICATION

(11) **EP 2 505 587 A1**
(43) Date of publication of application: **03.10.2012**
(21) Application number: 10833266.9
(22) Date of filing: 25.11.2010
(51) Int. Cl.: C07D 417/12, C07C 213/02, C07C 217/90, C07D 277/20, C07D 277/34, A61K 31/427, A61P 3/10, A61P 29/00, A61P 35/00, A61P 43/00

(54) **METHOD FOR MANUFACTURING A 6-SUBSTITUTED-1-METHYL-1H-BENZIMIDAZOLE DERIVATIVE, AND MANUFACTURING INTERMEDIATE FROM SAID METHOD**

(30) Priority: 26.11.2009 JP 2009269078
(71) Applicant: Daiichi Sankyo Company, Limited, Tokyo 103-8426 (JP)
(72) Inventor: IKEUCHI, Yutaka, Kanagawa 254-0014 (JP); SATOH, Chiharu, Kanagawa 254-0014 (JP); NUMAGAMI, Eiji, Kanagawa 254-0014 (JP); NIHEI, Satoru, Kanagawa 254-0014 (JP); KAJINO, Hisaki, Kanagawa 254-0014 (JP)
(74) Representative: Fairbairn, Angus Chisholm
(86) International application number: PCT/JP2010/071014
(87) International publication number: WO 2011/065420

(57) **Abstract**

It is intended to provide a novel method for manufacturing a 6-substituted 1-methyl-1H-benzimidazole derivative and an intermediate of said methods.

[Solution]

A method for manufacturing a 6-substituted 1-methyl-1H-benzimidazole derivative represented by the following general formula (IV) 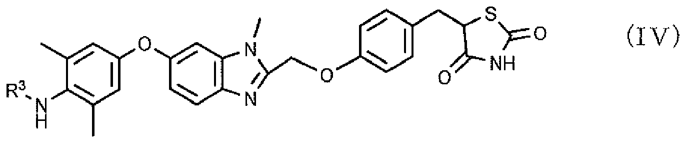 [wherein R³ represents a hydrogen atom or a C₁-C₄ alkyl group], comprising manufacturing a 4-substituted N²-methylbenzene-1,2-diamine derivative from a particular 5-substituted N-methyl-2-nitroaniline derivative, subsequently reacting the resulting compound with {4-[(2,4-dioxo-1,3-thiazolidin-5-yl)methyl]phenoxy}acetic acid or an acid chloride or a mixed acid anhydride thereof, and subsequently subjecting the resulting compound to intramolecular dehydration condensation.

## Description

### Technical Field

The present invention relates to a method for manufacturing a 6-substituted 1-methyl-1H-benzimidazole derivative that has an excellent ability to activate peroxisome proliferator-activated receptor (PPAR) γ and has an excellent anticancer effect, and an intermediate of said method.

### Background Art

It is known that 6-substituted 1-methyl-1H-benzimidazole derivatives have an excellent insulin resistance-improving effect, hypoglycemic effect, antiinflammatory effect, aldose reductase inhibitory effect, 5-lipoxygenase inhibitory effect, lipid peroxide formation inhibitory effect, PPAR-activating effect, leukotriene antagonistic effect, adipogenesis-promoting effect, cancer cell growth inhibitory effect, and calcium antagonistic effect (Patent Literatures 1, 2, 3, and 4). It is known that these 6-substituted 1-methyl-1H-benzimidazole derivatives can be synthesized by the condensation reaction between 4-substituted N²-methylbenzene-1,2-diamines having a protected N-methylamino group and corresponding carboxylic acid derivatives, followed by deprotection and intramolecular dehydration reaction (Patent Literatures 2, 3, 4, 5, and 6) .
However, if 6-substituted 1-methyl-1H-benzimidazole derivatives of high quality can be produced at high yields without protecting the amino groups of 4-substituted N²-methylbenzene-1,2-diamines, two steps for the introduction of protective groups and deprotection of the protective groups, can be eliminated and the reagents for the introduction of protective groups and deprotection of the protective groups can be reduced. This can provide a more industrially advantageous manufacturing method. A method comprising reacting 4-substituted N²-methylbenzene-1,2-diamines with carboxylic acid esters (Patent Literature 1) or a method comprising reacting 5-substituted N-methyl-2-nitroanilines with acid chlorides to obtain corresponding amide forms and further reducing the nitro groups (Patent Literature 7) is known as a method for manufacturing a 6-substituted 1-methyl-1H-benzimidazole derivative without the use of protective groups for the amino groups of the 4-substituted N²-methylbenzene-1,2-diamines. However, each of these methods was insufficient as an industrial manufacturing method due to low yields of the 6-substituted 1-methyl-1H-benzimidazole derivative of interest.

### Prior Art Literatures

### Patent Literatures

Patent Literature 1: Japanese Patent No. 2976885 (U.S.
Patent No. 5886014, European Patent No. 745600)
Patent Literature 2: Japanese Patent No. 3488099 (U.S.
Patent No. 6432993, European Patent No. 1022272)
Patent Literature 3: Japanese Patent Laid-Open No. 2000-351777 (pamphlet of International Publication No. WO 00/61582)
Patent Literature 4: Japanese Patent No. 4197387 (U.S.
Patent No. 6562849, European Patent Publication No. 1167366)
Patent Literature 5: Japanese Patent Laid-Open No. 2001-97954
Patent Literature 6: Japanese Patent Laid-Open No. 2001-72671 (U.S. Patent No. 6525220, European Patent No. 1191019)
Patent Literature 7: Japanese Patent Laid-Open No. 2006-225382 (U.S. Patent Publication No. 2008/0103185, European Patent Publication No. 1894929)
Patent Literature 8: Japanese Patent Laid-Open No. 2006-124375 (U.S. Patent Publication No. 2009/0023929, European Patent Publication No. 1798216)

### Summary of the Invention

### Problems to be solved by the Invention

The present inventors have found a method for manufacturing a highly pure 4-substituted N²-methylbenzene-1,2-diamine derivative at high yields without protecting the N-methylamino group, a method for selectively reacting one amino group of a 4-substituted N²-methylbenzene-1,2-diamine derivative with a carboxylic acid derivative, a method for further forming a 6-substituted 1-methyl-1H-benzimidazole derivative at high yields by the intramolecular dehydration reaction of the obtained amide form, and a novel synthetic intermediate of these manufacturing methods, and thus have completed the present invention.

### Means for solving the Problems

Thus, the present invention is directed to:
(1) a method for manufacturing a 6-substituted 1-methyl-1H-benzimidazole derivative represented by the following general formula (IV) or a salt thereof, or a hydrate thereof, comprising subjecting a compound represented by the following general formula (II) to intramolecular dehydration condensation in the presence of acid, and, if necessary, deprotecting a protective group for the nitrogen atom:

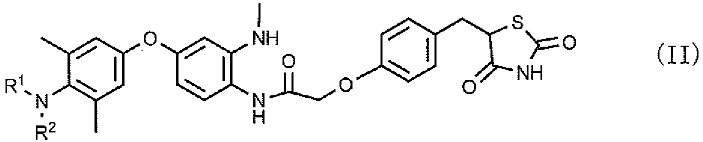

wherein R¹ represents a hydrogen atom, a C₁-C₄ alkyl group, or a protective group for the nitrogen atom; and R² represents a hydrogen atom or a protective group for the nitrogen atom, and

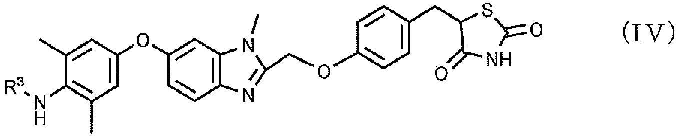

wherein R³ represents a hydrogen atom or a C₁-C₄ alkyl group;
(2) the manufacturing method according to (1) described above, wherein the acid is hydrochloric acid, R¹ represents a hydrogen atom, and R² represents a hydrogen atom or a t-butoxycarbonyl group;
(3) a compound represented by the following general formula (II):

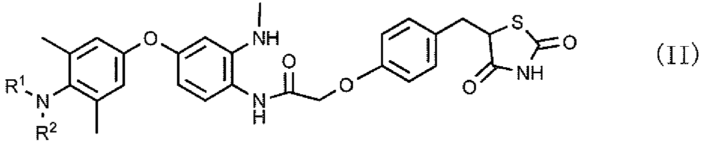

wherein R¹ represents a hydrogen atom, a C₁-C₄ alkyl group, or a protective group for the nitrogen atom; and R² represents a hydrogen atom or a protective group for the nitrogen atom;
(4) the compound according to (3) described above, wherein R¹ represents a hydrogen atom, and R² represents a hydrogen atom or a t-butoxycarbonyl group;
(5) a method for manufacturing a compound represented by the following general formula (II), comprising reacting a 4-substituted N²-methylbenzene-1,2-diamine derivative represented by the following general formula (I) with {4-[(2,4-dioxo-1,3-thiazolidin-5-yl)methyl]phenoxy}acetic acid in the presence of a condensing agent:

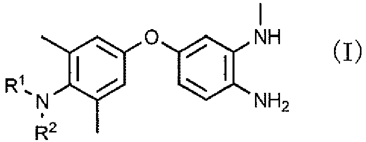

wherein R¹ represents a hydrogen atom, a C₁-C₄ alkyl group, or a protective group for the nitrogen atom; and R² represents a hydrogen atom or a protective group for the nitrogen atom, and

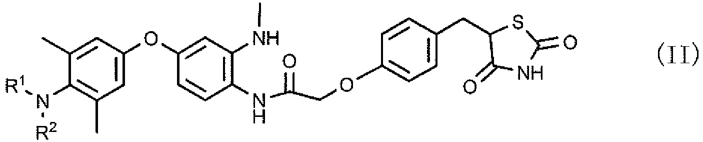

wherein R¹ represents a hydrogen atom, a C₁-C₄ alkyl group, or a protective group for the nitrogen atom; and R² represents a hydrogen atom or a protective group for the nitrogen atom;
(6) the manufacturing method according to (5) described above, wherein R¹ represents a hydrogen atom, and R² represents a hydrogen atom or a t-butoxycarbonyl group;
(7) a method for manufacturing a compound represented by the following general formula (II), comprising reacting a 4-substituted N²-methylbenzene-1,2-diamine derivative represented by the following general formula (I) with an acid chloride or a mixed acid anhydride of {4-[(2,4-dioxo-1,3-thiazolidin-5-yl)methyl]phenoxy}acetic acid:

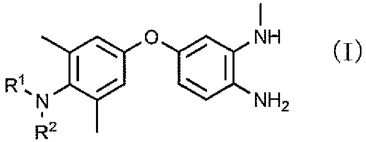

wherein R¹ represents a hydrogen atom, a C₁-C₄ alkyl group, or a protective group for the nitrogen atom; and R² represents a hydrogen atom or a protective group for the nitrogen atom, and

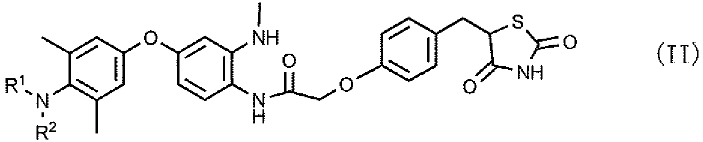

wherein R¹ represents a hydrogen atom, a C₁-C₄ alkyl group, or a protective group for the nitrogen atom; and R² represents a hydrogen atom or a protective group for the nitrogen atom;
(8) the manufacturing method according to (7) described above, wherein R¹ represents a hydrogen atom, and R² represents a hydrogen atom or a t-butoxycarbonyl group;
(9) a 4-substituted N²-methylbenzene-1,2-diamine derivative represented by the following general formula (I) or a salt thereof:

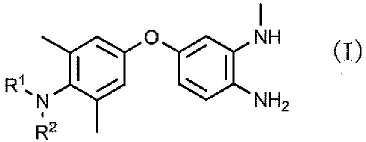

wherein R¹ represents a hydrogen atom, a C₁-C₄ alkyl group, or a protective group for the nitrogen atom; and R² represents a hydrogen atom or a protective group for the nitrogen atom;
(10) the 4-substituted N²-methylbenzene-1,2-diamine derivative according to (9) described above or a salt thereof, wherein R¹ represents a hydrogen atom, and R² represents a hydrogen atom or a t-butoxycarbonyl group;
(11) a method for manufacturing a 4-substituted N²-methylbenzene-1,2-diamine derivative represented by the following general formula (I), comprising reducing a 5-substituted N-methyl-2-nitroaniline derivative represented by the following general formula (III) by hydrogenation:

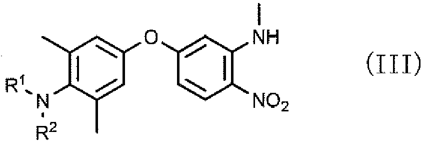

wherein R¹ represents a hydrogen atom, a C₁-C₄ alkyl group, or a protective group for the nitrogen atom; and R² represents a hydrogen atom or a protective group for the nitrogen atom, and

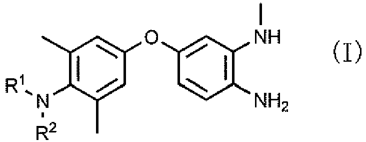

wherein R¹ represents a hydrogen atom, a C₁-C₄ alkyl group, or a protective group for the nitrogen atom; and R² represents a hydrogen atom or a protective group for the nitrogen atom;
(12) the manufacturing method according to (11), wherein R¹ represents a hydrogen atom, and R² represents a hydrogen atom or a t-butoxycarbonyl group; and (13) a method for manufacturing a 6-substituted 1-methyl-1H-benzimidazole derivative represented by the following general formula (I) or a salt thereof, or a hydrate thereof, comprising
   reducing a 5-substituted N-methyl-2-nitroaniline derivative represented by the following general formula (III) by hydrogenation to produce a 4-substituted N²-methylbenzene-1,2-diamine derivative represented by the following general formula (I),
   subsequently reacting the 4-substituted N²-methylbenzene-1,2-diamine derivative represented by the formula (I) with {4-[(2,4-dioxo-1,3-thiazolidin-5-yl)methyl]phenoxy}acetic acid in the presence of a condensing agent or reacting the 4-substituted N²-methylbenzene-1,2-diamine derivative represented by the formula (I) with an acid chloride or a mixed acid anhydride of {4-[(2,4-dioxo-1,3-thiazolidin-5-yl)methyl]phenoxy}acetic acid to produce a compound represented by the following general formula (II),
   subsequently subjecting the compound represented by the formula (II) to intramolecular dehydration condensation in the presence of an acid, and, if necessary, deprotecting a protective group for the nitrogen atom:

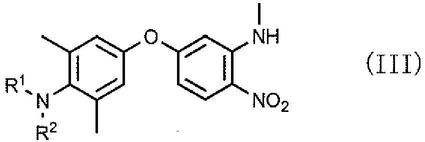

wherein R¹ represents a hydrogen atom, a C₁-C₄ alkyl group, or a protective group for the nitrogen atom; and R² represents a hydrogen atom or a protective group for the nitrogen atom,

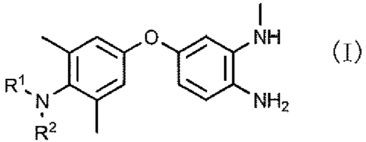

wherein R¹ represents a hydrogen atom, a C₁-C₄ alkyl group, or a protective group for the nitrogen atom; and R² represents a hydrogen atom or a protective group for the nitrogen atom,

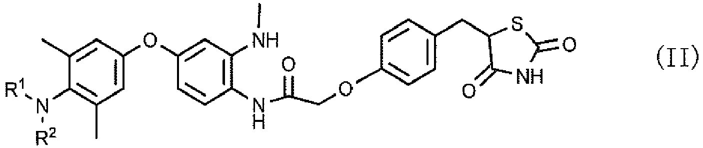

wherein R¹ represents a hydrogen atom, a C₁-C₄ alkyl group, or a protective group for the nitrogen atom; and R² represents a hydrogen atom or a protective group for the nitrogen atom, and

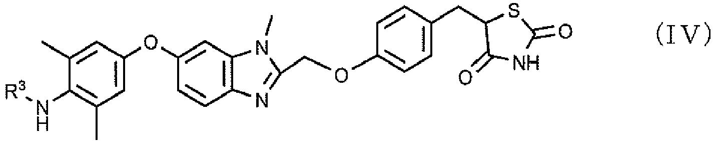

wherein R³ represents a hydrogen atom or a C₁-C₄ alkyl group.
In the present invention, the C₁-C₄ alkyl group is a linear or branched alkyl group having 1 to 4 carbon atoms and is, for example, a methyl group, an ethyl group, a propyl group, a butyl group, an isopropyl group, an isobutyl group, an s-butyl group, or a t-butyl group. The C₁-C₄ alkyl group is preferably a methyl group, an ethyl group, a propyl group, a butyl group, or an isobutyl group, more preferably a methyl group.

In the present invention, the "protective group for the nitrogen atom" means a protective group for a nitrogen atom usually used in organic synthesis. Examples of such a group can include an aliphatic acyl group, an aromatic acyl group, a silyl group, an alkoxycarbonyl group, an alkenyloxycarbonyl group, and an aralkyl group.

The aliphatic acyl groups are, for example, unsaturated alkylcarbonyl groups such as acryloyl, propioloyl, methacryloyl, crotonoyl, isocrotonoyl, and (E)-2-methyl-2-butenoyl.

The aromatic acyl groups are, for example, nitrated arylcarbonyl groups such as 4-nitrobenzoyl and 2-nitrobenzoyl, lower alkoxycarbonylated arylcarbonyl groups such as 2-(methoxycarbonyl)benzoyl, or arylated arylcarbonyl groups such as 4-phenylbenzoyl.

The silyl groups are, for example, tri-lower alkylsilyl groups such as trimethylsilyl, triethylsilyl, isopropyldimethylsilyl, t-butyldimethylsilyl, methyldiisopropylsilyl, methyl-di-t-butylsilyl, and triisopropylsilyl; or tri-lower alkylsilyl groups substituted by 1 or 2 aryl groups, such as diphenylmethylsilyl, diphenylbutylsilyl, diphenylisopropylsilyl, and phenyldiisopropylsilyl.

The alkoxycarbonyl groups are, for example, lower alkoxycarbonyl groups having 1 to 6 carbon atoms, such as methoxycarbonyl, ethoxycarbonyl, propoxycarbonyl, butoxycarbonyl, s-butoxycarbonyl, t-butoxycarbonyl, and isobutoxycarbonyl.

The alkenyloxycarbonyl groups are, for example, lower alkenyloxycarbonyl having 3 to 6 carbon atoms, such as allyloxycarbonyl and methallyloxycarbonyl.

The aralkyl groups are, for example, aralkyl groups having an aryl ring substituted by 1 to 3 groups selected from lower alkyl, lower alkoxy, nitro, halogen, and cyano group, such as 4-methylbenzyl, 2,4,6-trimethylbenzyl, 3,4,5-trimethylbenzyl, 4-methoxybenzyl, 2-nitrobenzyl, 4-nitrobenzyl, 4-chlorobenzyl, 4-bromobenzyl, and 4-cyanobenzyl.

Among these, the alkoxycarbonyl groups are preferred, and the t-butoxycarbonyl group is particularly preferred.

Where a 4-substituted N²-methylbenzene-1,2-diamine derivative represented by the general formula (I) or a 6-substituted 1-methyl-1H-benzimidazole derivative represented by the general formula (IV) according to the present invention has a basic group such as an amino group, the derivative can be converted into a salt by reacting it with an acid. The present invention, a salt or a pharmacologically acceptable salt refers to such salts.

The salt includes: inorganic acid salts such as hydrochloride, hydrobromide, hydroiodide, sulfate, nitrate, phosphate, and borate; sulfonates such as methanesulfonate, ethanesulfonate, benzenesulfonate, p-toluenesulfonate, and camphorsulfonate; organic carboxylates such as formate, acetate, propionate, oxalate, hydroxyacetate, citrate, tartrate, succinate, maleate, benzoate, salicylate, fumarate, and phthalate; etc. The salt is preferably hydrochloride, hydrobromide, sulfate, phosphate, methanesulfonate, benzenesulfonate, p-toluenesulfonate, oxalate, or tartrate, more preferably hydrochloride, hydrobromide, sulfate, methanesulfonate, or p-toluenesulfonate, further preferably hydrochloride.

Examples of specific compounds of the general formula (I) or (II) of the present invention can include compounds described in the table below. However, the compound of the present invention represented by the general formula (I) or (II) is not limited to these compounds.

Abbreviations in the table are as follows:
- Me:: Methyl
- Et:: Ethyl
- Pr:: Propyl
- iPr:: Isopropyl
- Bu:: n-Butyl
- iBu:: Isobutyl
- sBu:: s-Butyl
- tBu:: t-Butyl
- Boc:: t-Butoxycarbonyl

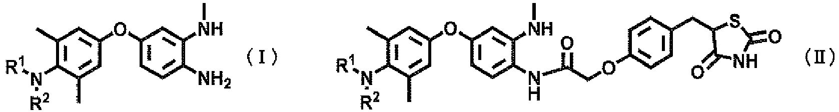

**(Table 1)**

| Compound No. | Compound No. | R¹ | R² |
|---|---|---|---|
| (I)-1 | (II)-1 | H | H |
| (I)-2 | (II)-2 | Me | H |
| (I)-3 | (II)-3 | Et | H |
| (I)-4 | (II)-4 | Pr | H |
| (I)-5 | (II)-5 | iPr | H |
| (I)-6 | (II)-6 | Bu | H |
| (I)-7 | (II)-7 | iBu | H |
| (I)-8 | (II) -8 | sBu | H |
| (I)-9 | (II)-9 | tBu | H |
| (I)-10 | (II) -10 | Boc | H |
| (I)-11 | (II) -11 | H | Boc |
| (I)-12 | (II)-12 | Me | Boc |
| (I)-13 | (II)-13 | Et | Boc |
| (I)-14 | (II)-14 | Pr | Boc |
| (I)-15 | (II)-15 | iPr | Boc |
| (I)-16 | (II)-16 | Bu | Boc |
| (I)-17 | (II)-17 | iBu | Boc |
| (I)-18 | (II)-18 | sBu | Boc |
| (I)-19 | (II)-19 | tBu | Boc |

The preferred compounds of the compound represented by the general formula (I) of the present invention include the compounds of Compound Nos. (I)-1, (I)-2, (I)-3, (I)-4, (I)-6, (I)-7, (I)-11, and (I)-12, more preferably (I)-1, (I)-2, and (I)-11, further preferably (I)-1 and (I) -11, still further preferably (I)-1.

The compound of the present invention represented by the general formula (II) is preferably the exemplary compounds of Compound Nos. (II)-1, (II)-2, (II)-3, (II)-4, (II)-6, (II)-7, (II)-11, and (II)-12, more preferably (II)-1, (II)-2, and (II)-11, further preferably (II)-1 and (II) -11, still further preferably (II)-1.

### Effects of the Invention

The present invention provides novel intermediates (I) and (II) for manufacturing a 6-substituted 1-methyl-1H-benzimidazole derivative represented by the general formula (IV), which is a pharmaceutically active ingredient known in the art. The present invention further provides a manufacturing method of the synthetic intermediate (I) or (II) and a manufacturing method of the pharmaceutically active ingredient 6-substituted 1-methyl-1H-benzimidazole derivative at high yields and high purity, which is inexpensive and suitable for large-scale production.

### Brief Description of Drawing

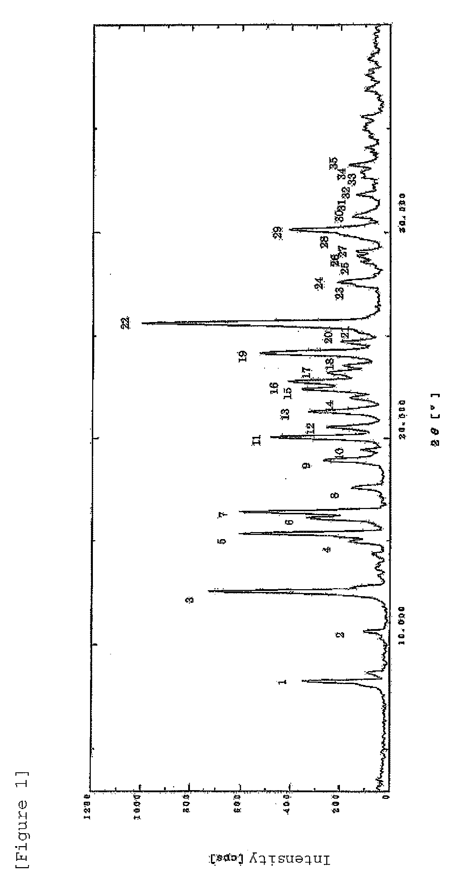

[Figure 1] Figure 1 shows a powder X-ray diffraction pattern of the crystalline form obtained in Example 11. In the diagram, the vertical axis represents diffraction intensity in units of count/second (cps), and the horizontal axis represents values of diffraction angle 2θ.

### Description of Embodiments

Hereinafter, a method for manufacturing synthetic intermediate (I) or a salt thereof, synthetic intermediate (II), or final compound (IV) of interest or a salt thereof, or a hydrate thereof, of the present invention will be described in detail.

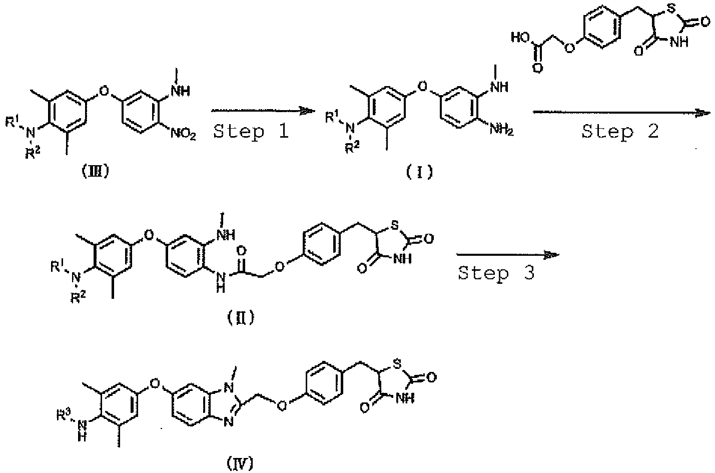

In the formula, R¹, R², and R³ are as defined above.

The method of the present invention comprises
Step 1 of reducing the nitro group of a 5-substituted N-methyl-2-nitroaniline derivative (III) by catalytic hydrogenation to produce a novel 4-substituted N²-methylbenzene-1,2-diamine derivative (I) or a salt thereof,
Step 2 of reacting the 4-substituted N²-methylbenzene-1,2-diamine derivative (I) or the salt thereof with 4-[(2,4-dioxothiazolidin-5-yl)methyl]phenoxyacetic acid in the presence of a condensing agent or reacting the 4-substituted N²-methylbenzene-1,2-diamine derivative (I) or the salt thereof with an acid chloride or a mixed acid anhydride of 4-[(2,4-dioxothiazolidin-5-yl)methyl]phenoxyacetic acid to produce a novel amide compound (II), and
Step 3 of subjecting the novel amide compound (II) to dehydration condensation in the presence of an acid to obtain a 6-substituted 1-methyl-1H-benzimidazole derivative (IV) or a salt thereof.
Hereinafter, each step will be described specifically.

### (Step 1)

This step is the step of reducing the nitro group of a 5-substituted N-methyl-2-nitroaniline derivative (III) by catalytic hydrogenation in the presence of a catalyst in a solvent to manufacture a 4-substituted N²-methylbenzene-1,2-diamine derivative (I).

In this context, the starting material 5-substituted N-methyl-2-nitroaniline derivative (III) is a compound known in the art, which is disclosed in the pamphlet of International Publication No. WO 2006/035685, and can be synthesized easily by a manufacturing method described in the pamphlet of WO 2006/035685.

The catalyst used in this step is not particularly limited as long as it is usually used in hydrogenation. Examples of such a catalyst include palladium carbon catalysts, palladium hydroxide, platinum carbon catalysts, and Raney nickel. Preferred examples thereof include palladium carbon catalysts, palladium hydroxide, and platinum carbon catalysts, more preferably palladium carbon catalysts.

The amount of the catalyst used in this step is not particularly limited and is usually 0.000001 equivalents to 1 equivalent, preferably 0.00001 equivalents to 0.5 equivalents, further preferably 0.0001 equivalents to 0.1 equivalents, with respect to the 5-substituted N-methyl-2-nitroaniline derivative (III).

Since the 4-substituted N²-methylbenzene-1,2-diamine derivative (I) obtained in this step is susceptible to oxidation, an antioxidant may be added into the reaction system to prevent this oxidation. When an antioxidant is added thereto, the antioxidant used is not particularly limited as long as it does not influence the reaction and does not react with the starting material, the product, or the solvent, or the like. Preferably, 2,6-di-t-butyl-4-methylphenol is used. The amount of the antioxidant used is not particularly limited. The antioxidant is usually used at 0.0001 equivalents to 1 equivalent, preferably 0.001 equivalents to 0.1 equivalent, with respect to the 5-substituted N-methyl-2-nitroaniline derivative (III) used.

The hydrogen pressure in this step is not particularly limited and is usually 1 atmospheric pressure to 30 atmospheric pressures, preferably 1 atmospheric pressure to 10 atmospheric pressures, more preferably 1 atmospheric pressure to 5 atmospheric pressures.

The reaction in this step is usually performed in a solvent. The solvent is not particularly limited as long as it does not react under reaction conditions for carrying out this step. Such a solvent is, for example, water; alcohols such as methanol, ethanol, propanol, isopropyl alcohol, butanol, and isobutyl alcohol; ethers such as diethyl ether, diisopropyl ether, dimethoxyethane, dibutyl ether, tetrahydrofuran, cyclopentyl methyl ether, and dioxane; esters such as methyl formate, ethyl formate, methyl acetate, ethyl acetate, propyl acetate, isopropyl acetate, and butyl acetate; nitriles such as acetonitrile, propionitrile, and benzonitrile; amides such as dimethylformamide, dimethylacetamide, and N,N-dimethylimidazolidinone; hydrocarbons such as pentane, hexane, heptane, benzene, and toluene; or a mixture thereof. The solvent is preferably water, methanol, ethanol, propanol, isopropyl alcohol, tetrahydrofuran, ethyl acetate, acetonitrile, dimethylformamide, dimethylacetamide, or a mixture thereof, more preferably water, methanol, ethanol, propanol, isopropyl alcohol, tetrahydrofuran, ethyl acetate, or a mixture thereof.

The reaction temperature in this step is not particularly limited and is usually -20°C to 200°C, preferably -20°C to 120°C, more preferably 0°C to 80°C.

The reaction time in this step differs depending on reaction conditions such as the starting material 5-substituted N-methyl-2-nitroaniline (III) itself, the solvent used, the concentration, the number of equivalents of the catalyst used, the hydrogen pressure, and the reaction temperature and is usually 5 minutes to 5 days, preferably 10 minutes to 2 days, more preferably 30 minutes to 24 hours.

After completion of the reaction in this step, the catalyst is removed by filtration or the like, and then, a solution of the 4-substituted N²-methylbenzene-1,2-diamine derivative (I) of interest can be obtained. The obtained solution of the 4-substituted N²-methylbenzene-1,2-diamine derivative (I) may be used directly in the next step when the solvent neither reacts under the reaction conditions of the next step nor inhibits the reaction. Moreover, the 4-substituted N²-methylbenzene-1,2-diamine derivative (I) may be isolated by concentration. Moreover, the obtained 4-substituted N²-methylbenzene-1,2-diamine derivative (I) can also be isolated by procedures such as filtration when it is deposited as crystals. The isolated 4-substituted N²-methylbenzene-1,2-diamine derivative (I) can also be further purified by usual purification procedures such as recrystallization, distillation, and column chromatography. The isolated 4-substituted N²-methylbenzene-1,2-diamine derivative (I) may be used in the next step after drying or can also be used in the next step without drying.

Both the reaction solution of the 4-substituted N²-methylbenzene-1,2-diamine derivative (I) and the isolated 4-substituted N²-methylbenzene-1,2-diamine derivative (I) may be allowed to form a salt by the addition of an acid thereto.

When the 4-substituted N²-methylbenzene-1,2-diamine derivative (I) is allowed to form a salt, the acid used is not particularly limited as long as it forms a salt. Examples of such an acid include: inorganic acids such as hydrogen chloride, hydrogen bromide, hydrogen iodide, hydrochloric acid, hydrobromic acid, sulfuric acid, nitric acid, phosphoric acid, and boric acid; sulfonic acids such as methanesulfonic acid, ethanesulfonic acid, benzenesulfonic acid, p-toluenesulfonic acid, and camphorsulfonic acid; and organic carboxylic acids such as formic acid, acetic acid, propionic acid, oxalic acid, hydroxyacetic acid, citric acid, tartaric acid, succinic acid, maleic acid, benzoic acid, salicylic acid, fumaric acid, and phthalic acid. The acid is preferably hydrogen chloride, hydrochloric acid, hydrobromic acid, sulfuric acid, phosphoric acid, methanesulfonic acid, benzenesulfonic acid, p-toluenesulfonic acid, oxalic acid, or tartaric acid, more preferably hydrochloric acid, hydrobromic acid, sulfuric acid, methanesulfonic acid, or p-toluenesulfonic acid.

When the 4-substituted N²-methylbenzene1,2-diamine derivative (I) is allowed to form a salt, the amount of the acid used is equal to or more than the number of equivalents necessary for the salt to be formed, with respect to the 4-substituted N²-methylbenzene-1,2-diamine derivative (I). Thus, a tribasic acid such as boric acid or phosphoric acid at 1/3 equivalent or more, a dibasic acid such as sulfuric acid, oxalic acid, maleic acid, or tartaric acid at 0.5 equivalent or more, or a monobasic acid such as hydrogen chloride, hydrochloric acid, nitric acid, or methanesulfonic acid at 1 equivalent or more is used.

When the 4-substituted N²-methylbenzene-1,2-diamine derivative (I) is allowed to form a salt, the salt formation is usually performed in a solvent. The solvent is not particularly limited as long as it does not react under reaction conditions for carrying out this step. Such a solvent is, for example, water; alcohols such as methanol, ethanol, propanol, isopropyl alcohol, butanol, and isobutyl alcohol; ethers such as diethyl ether, diisopropyl ether, dimethoxyethane, dibutyl ether, tetrahydrofuran, cyclopentyl methyl ether, and dioxane; esters such as methyl formate, ethyl formate, methyl acetate, ethyl acetate, propyl acetate, isopropyl acetate, and butyl acetate; nitriles such as acetonitrile, propionitrile, and benzonitrile; amides such as dimethylformamide, dimethylacetamide, and N,N-dimethylimidazolidinone; hydrocarbons such as pentane, hexane, heptane, benzene, and toluene; or a mixture thereof. The solvent is preferably water, methanol, ethanol, propanol, isopropyl alcohol, tetrahydrofuran, ethyl acetate, acetonitrile, dimethylformamide, dimethylacetamide, or a mixture thereof, more preferably water, methanol, ethanol, propanol, isopropyl alcohol, tetrahydrofuran, ethyl acetate, or a mixture thereof.

When the 4-substituted N²-methylbenzene-1,2-diamine derivative (I) is allowed to form a salt, the salt formation temperature is not particularly limited and is usually -20°C to 200°C, preferably -20°C to 120°C, more preferably 0°C to 80°C.

When the 4-substituted N²-methylbenzene-1,2-diamine derivative (I) is allowed to form a salt, the salt formation time varies depending on reaction conditions such as the starting material 5-substituted N-methyl-2-nitroaniline derivative (III) itself, the solvent used, the concentration, the number of equivalents of the acid used, and the reaction temperature and is usually 5 minutes to 5 days, preferably 10 minutes to 2 days, more preferably 30 minutes to 24 hours.

In case the 4-substituted N²-methylbenzene-1,2-diamine derivative (I) is isolated as a salt, the obtained salt can be isolated by separation procedures such as filtration when it is deposited as crystals. The salt of the 4-substituted N²-methylbenzene-1,2-diamine derivative (I) isolated as crystals can also be further purified by recrystallization. Moreover, the isolated salt of the 4-substituted N²-methylbenzene-1,2-diamine derivative (I) may be used in the next step without drying, or its dried product may be used in the next step.

### (Step 2)

This step is generally called amidation and is the step of (i) reacting the 4-substituted N²-methylbenzene-1,2-diamine derivative (I) with {4-[(2,4-dioxo-1,3-thiazolidin-5-yl)methyl]phenoxy}acetic acid using a condensing agent in the presence of a base in a solvent or (ii) forming an acid chloride or a mixed acid anhydride of {4-[(2,4-dioxo-1,3-thiazolidin-5-yl)methyl]phenoxy}acetic acid and reacting this acid chloride or mixed acid anhydride with the 4-substituted N²-methylbenzene-1,2-diamine derivative (I) or a salt thereof in the presence of a base in a solvent to thereby produce a novel amide compound (II). In this context, the starting compound 4-substituted N²-methylbenzene-1,2-diamine derivative (I) as a salt may be used in this step. As the salt, a salt of the 4-substituted N²-methylbenzene-1,2-diamine derivative (I) as described above in (Step 1) can be used.

When {4-[(2,4-dioxo-1,3-thiazolidin-5-yl)methyl]phenoxy}acetic acid is reacted in the presence of a condensing agent in this step, the condensing agent used is not particularly limited as long as it is a condensing agent generally used in amidation reactions. Examples of such a condensing agent include dicyclohexylcarbodiimide (DCC), diisopropylcarbodiimide (DTPC), N-ethyl-N'-3-dimethylaminopropyl carbodiimide, benzotriazol-1-yl-tris(dimethylamino)phosphonium hexafluorophosphate (BOP), methylphosphonic acid cyclic anhydride, ethylphosphonic acid cyclic anhydride, propylphosphonic acid cyclic anhydride, butylphosphonic acid cyclic anhydride, polyphosphoric acid ethyl ester (PPE), polyphosphoric acid trimethylsilyl ester (PPSE), dimethylphosphoryl cyanide, diethylphosphoryl cyanide, dibutylphosphoryl cyanide, diphenylphosphoryl azide (DPPA), ditolylphosphoryl azide, and dinaphthylphosphoryl azide. The condensing agent is preferably dicyclohexylcarbodiimide, N-ethyl-N'-3-dimethylaminopropyl carbodiimide, ethylphosphonic acid cyclic anhydride, propylphosphonic acid cyclic anhydride, polyphosphoric acid silyl ester (PPSE), or diethylphosphoryl cyanide, more preferably N-ethyl-N'-3-dimethylaminopropyl carbodiimide or propylphosphonic acid cyclic anhydride.

The amount of the condensing agent used is not particularly limited as long as it is equal to or more than 1 equivalent with respect to {4-[(2,4-dioxo-1,3-thiazolidin-5-yl)methyl]phenoxy}acetic acid. The condensing agent is usually used at 1 equivalent to 3 equivalents, preferably 1 equivalent to 1.5 equivalents, more preferably 1 equivalent to 1.2 equivalents.

When an acid chloride of {4-[(2,4-dioxo-1,3-thiazolidin-5-yl)methyl]phenoxy}acetic acid is used in this step, the acid chloride of {4-[(2,4-dioxo-1,3-thiazolidin-5-yl)methyl]phenoxy}acetic acid can be produced by a method described in, for example, the pamphlet of International Publication No. WO 2006/35685 (Patent Literature 7).

When a mixed acid anhydride of {4-[(2,4-dioxo-1,3-thiazolidin-5-yl)methyl]phenoxy}acetic acid is used in this step, the mixed acid anhydride of {4-[(2,4-dioxo-1,3-thiazolidin-5-yl)methyl]phenoxy}acetic acid can be produced by reacting {4-[(2,4-dioxo-1,3-thiazolidin-5-yl)methyl]phenoxy}acetic acid with an acid chloride in the presence of a base in a solvent.

Carboxylic acid chlorides such as pivaloyl chloride and (2,2-dimethylbutanoyl) chloride; or chlorocarbonic acid esters such as methyl chlorocarbonate, ethyl chlorocarbonate, propyl chlorocarbonate, isopropyl chlorocarbonate, butyl chlorocarbonate, isobutyl chlorocarbonate, s-butyl chlorocarbonate, t-butyl chlorocarbonate, and phenyl chlorocarbonate are used as the acid chloride used for manufacturing the mixed acid anhydride of {4-[(2,4-dioxo-1,3-thiazolidin-5-yl)methyl]phenoxy}acetic acid. The acid chloride is preferably pivaloyl chloride, methyl chlorocarbonate, ethyl chlorocarbonate, propyl chlorocarbonate, or isopropyl chlorocarbonate, more preferably pivaloyl chloride or isopropyl chlorocarbonate.

The amount of the acid chloride used is usually 0.8 equivalent to 1.2 equivalents, preferably 0.9 equivalent to 1.1 equivalents, more preferably 0.95 equivalent to 1.05 equivalents, with respect to {4-[(2,4-dioxo-1,3-thiazolidin-5-yl)methyl]phenoxy}acetic acid.

The base used for manufacturing the mixed acid anhydride of {4-[2,4-dioxo-1,3-thiazolidin-5-yl)methyl]phenoxy}acetic acid is an organic amine such as trimethylamine, triethylamine, diisopropylethylamine, tripropylamine, triisopropylamine, tributylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU). The base is preferably triethylamine, diisopropylethylamine, tripropylamine, tributylamine, or N-methylmorpholine, more preferably triethylamine, tributylamine, or N-methylmorpholine.

The amount of the base used is usually 0.8 equivalent to 1.2 equivalents, preferably 0.9 equivalent to 1.1 equivalents, more preferably 0.95 equivalent to 1.05 equivalents, with respect to 4-[(2,4-dioxothiazolidin-5-yl)methyl]phenoxyacetic acid.

The production of the mixed acid anhydride of {4-[(2,4-dioxo-1,3-thiazolidin-5-yl)methyl]phenoxy}acetic acid is usually performed in a solvent. The solvent is not particularly limited as long as it does not react under the production conditions of the mixed acid anhydride. Such solvents are ethers such as diethyl ether, diisopropyl ether, dimethoxyethane, dibutyl ether, tetrahydrofuran, cyclopentyl methyl ether, and dioxane; esters such as methyl formate, ethyl formate, methyl acetate, ethyl acetate, propyl acetate, isopropyl acetate, and butyl acetate; nitriles such as acetonitrile, propionitrile, and benzonitrile; amides such as dimethylformamide, dimethylacetamide, and N,N-dimethylimidazolidinone; hydrocarbons such as pentane, hexane, heptane, benzene, and toluene; or a mixture thereof. The solvent is preferably dibutyl ether, tetrahydrofuran, ethyl acetate, acetonitrile, or a mixture thereof, more preferably tetrahydrofuran.

The reaction temperature in the production of the mixed acid anhydride described above is usually -70°C to 10°C, preferably -50°C to 10°C, more preferably -30°C to 5°C.

The reaction time in the production of the mixed acid anhydride described above is not particularly limited and is usually 5 minutes to 24 hours, preferably 5 minutes to 12 hours, more preferably 5 minutes to 6 hours.

The mixed acid anhydride is usually used directly after production without being, for example, isolated or purified.

The amount of the {4-[(2,4-dioxo-1,3-thiazolidin-5-yl)methyl]phenoxy}acetic acid or the acid chloride or the mixed acid anhydride thereof used in this step is usually 0.8 equivalent to 1.2 equivalents, preferably 0.9 equivalent to 1.1 equivalents, more preferably 0.95 equivalent to 1.05 equivalents, with respect to the 4-substituted N²-methylbenzene-1,2-diamine derivative (I) or the salt thereof used.

A base is generally used in the amidation in this step. The base used is an organic amine such as trimethylamine, triethylamine, diisopropylethylamine, tripropylamine, triisopropylamine, tributylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), and 1,8-diazabicyclo[5.4.0]-7-undecene (DBU). The base is preferably triethylamine, diisopropylethylamine, tripropylamine, tributylamine, or N-methylmorpholine, more preferably triethylamine, tributylamine, or N-methylmorpholine.

When the 4-substituted N²-methylbenzene-1,2-diamine derivative (I) is used in this step, the amount of the base used in this step is usually 0.8 equivalent to 1.2 equivalents, preferably 0.9 equivalent to 1.1 equivalents, more preferably 0.95 equivalent to 1.05 equivalents, with respect to the 4-substituted N²-methylbenzene-1,2-diamine derivative (I) used. When a salt of the 4-substituted N²-methylbenzene-1,2-diamine derivative (I) is used in this step, an amount necessary for neutralizing the salt is further added.

In addition to the base, N-hydroxysuccinimide, 1-hydroxybenzotriazole, or 3-hydroxy-4-oxo-3,4-dihydro-1,2,3-benzotriazine, or the like may be added in this step to thereby further improve yields and reduce the reaction time. When N-hydroxysuccinimide, 1-hydroxybenzotriazole, or 3-hydroxy-4-oxo-3,4-dihydro-1,2,3-benzotriazine, or the like is added, its amount is usually 0.001 equivalent to 1 equivalent, preferably 0.01 equivalent to 1 equivalent, with respect to the {4-[(2,4-dioxo-1,3-thiazolidin-5-yl)methyl]phenoxy}acetic acid or the acid chloride or the mixed acid anhydride thereof used.

This step is usually performed in a solvent. The solvent is not particularly limited as long as it does not react under reaction conditions in this step. Such solvents are ethers such as diethyl ether, diisopropyl ether, dimethoxyethane, dibutyl ether, tetrahydrofuran, cyclopentyl methyl ether, and dioxane; esters such as methyl formate, ethyl formate, methyl acetate, ethyl acetate, propyl acetate, isopropyl acetate, and butyl acetate; nitriles such as acetonitrile, propionitrile, and benzonitrile; amides such as dimethylformamide, dimethylacetamide, and N,N-dimethylimidazolidinone; hydrocarbons such as pentane, hexane, heptane, benzene, and toluene; halogenated hydrocarbons such as dichloromethane, chloroform, and 1,2-dichloroethane; or a mixture thereof. The solvent is preferably dibutyl ether, tetrahydrofuran, ethyl acetate, acetonitrile, dichloromethane, or a mixture thereof, more preferably ethyl acetate, tetrahydrofuran, dichloromethane, or a mixture thereof.

The reaction temperature in this step is usually - 50°C to 20°C, preferably -30°C to 20°C, more preferably - 20°C to 105°C.

The reaction time in this step is not particularly limited and is usually 5 minutes to 24 hours, preferably 5 minutes to 12 hours, more preferably 5 minutes to 6 hours.

The novel amide compound (II) formed in this step may be used directly in the next step without being isolated or can also be isolated by usual post-treatment.

### (Step 3)

This step is the step of subjecting the novel amide compound (II) to intramolecular dehydration condensation in the presence of an acid in a solvent, and, if necessary, deprotecting a protective group for the nitrogen atom to thereby produce a 6-substituted 1-methyl-1H-benzimidazole derivative (IV) or a salt thereof, or a hydrate thereof.

The solvent used in this step is not particularly limited as long as it does not react with the amide compound (II) and it dissolves the amide compound (II) to some extent. Such solvents are, for example, water; alcohols such as methanol, ethanol, propanol, isopropyl alcohol, butanol, and isobutyl alcohol; ethers such as diethyl ether, diisopropyl ether, dimethoxyethane, dibutyl ether, tetrahydrofuran, cyclopentyl methyl ether, and dioxane; nitriles such as acetonitrile, propionitrile, and benzonitrile; amides such as dimethylformamide, dimethylacetamide, and N,N-dimethylimidazolidinone; hydrocarbons such as pentane, hexane, heptane, benzene, and toluene; halogenated hydrocarbons such as dichloromethane, chloroform, and 1,2-dichloroethane; or a mixture thereof. The solvent is preferably, water, nitriles, alcohols, ethers, and a mixture thereof, more preferably, water, acetonitrile, methanol, ethanol, tetrahydrofuran, dioxane, or a mixture thereof, particularly preferably a water-acetonitrile mixed solvent or a water-tetrahydrofuran mixed solvent.

Any of Broensted acids and Lewis acids may be used as the acid used in this step. Usually, a Broensted acid is used. The Broensted acid usually used in this step is not particularly limited and is, for example, an inorganic acid such as hydrogen chloride, hydrogen bromide, hydrochloric acid, bromic acid, sulfuric acid, nitric acid, phosphoric acid, and boric acid; or an organic acid such as formic acid, acetic acid, propionic acid, oxalic acid, succinic acid, maleic acid, hydroxyacetic acid, fumaric acid, citric acid, tartaric acid, benzoic acid, and salicylic acid. The Broensted acid is preferably hydrochloric acid, sulfuric acid, acetic acid, citric acid, or tartaric acid, more preferably hydrochloric acid or sulfuric acid, particularly preferably hydrochloric acid.

When the compound (IV) is produced as a salt in this step, an acid of the salt to be produced can also be used directly to form the corresponding salt.

The amount of the acid used in this step is not particularly limited as long as it is equal to or more than 1 equivalent with respect to the amide compound (II) used. The amount of the acid is preferably 1 equivalent to 15 equivalents, more preferably 2 equivalents to 8 equivalents.

When the amide compound (II) is dissolved in the solvent in this step, inorganic or organic poorly soluble impurities mixed in the compound (II) can be removed by performing filtration procedures before addition of the acid.

The reaction temperature in this step differs depending on the properties of the amide compound (II), the acid, and the solvent and is usually 0°C to 150°C, preferably 220°C to 100°C, more preferably 30°C to 80°C.

The reaction time in this step differs depending on the properties of the amide compound (II), the acid, and the solvent and is usually 30 minutes to 20 hours, preferably 1 hour to 10 hours.

In this step, protective groups for the nitrogen atom substituted on R¹ and/or R² include groups deprotected during the reaction described above. Groups that are not deprotected during the reaction are subjected to a deprotection reaction, if necessary. Usual deprotection reactions routinely used in organic synthesis (reaction described in, e.g., T.W. Greene et al., Protective Groups in Organic Synthesis, John Willey & Sons, Inc.) can be adopted as the deprotection reaction. Preferably, a deprotection reaction under neutral or acidic conditions is adopted.

After completion of the reaction in this step, the compound (IV) or the salt thereof, or the hydrate thereof can be isolated by collection procedures based on filtration when it is deposited as crystals. When the compound (IV) or the salt thereof, or the hydrate thereof does not form crystals, it can be isolated by usual post-treatment, followed by isolation procedures such as extraction procedures.
The obtained compound (IV) or salt thereof, or hydrate thereof may further be subjected to usual purification procedures such as recrystallization and column chromatography.

The recrystallization is achieved by a method usually used in the field of organic synthetic chemistry, such as (1) a method comprising dissolving the obtained compound (IV) or salt thereof, or hydrate thereof by heating, followed by cooling, (2) a method comprising after dissolution, concentrating the solution by distilling off the solvent, or (3) a method comprising dissolving the obtained compound (IV) or salt thereof, or hydrate thereof in a good solvent and depositing crystals by the addition of a poor solvent.

The method for purifying the obtained compound (IV) or salt thereof, or hydrate thereof can be performed according to the methods described in National Publication of International Patent Application No. 2010-517932 (pamphlet of International Publication No. WO 2008/099944).
The compound (IV) and the salt (particularly preferably hydrochloride) thereof, and the hydrate thereof exhibit an excellent ability to activate peroxisome proliferator-activated receptor (PPAR) γ, as disclosed in Japanese Patent Laid-Open No. 11-193276 (Patent Literature 2), the pamphlet of International Publication No. WO 2007/091622, etc.

The 6-substituted 1-methyl-1H-benzimidazole derivative of the present invention represented by the general formula (IV) or the salt thereof, or the hydrate thereof is useful as a pharmaceutical drug, particularly, a PPARγ activator and is also useful as a therapeutic or preventive agent for various cancer species as described above (anticancer pharmaceutical composition).

### Examples

Hereinafter, the present invention will be described more specifically with reference to Examples thereof. However, the scope of the present invention is not limited to these.

### (Example 1)

### 4-(4-Amino-3,5-dimethylphenoxy)-N²-methylbenzenediamine (Compound No: (I) -1, Step 1)

A 7.5% palladium carbon catalyst (0.74 g) was added to a solution of 2,6-dimethyl-4-[3-(methylamino)-4-nitrophenoxy]aniline (3.5 g) in methanol (35 mL). The inside of the system was degassed under reduced pressure, followed by nitrogen substitution. The inside of the system was further degassed under reduced pressure, followed by hydrogen substitution. The mixture was stirred for 4.5 hours under the same atmosphere as above (1 atmospheric pressure). The reaction mixture was filtered and then concentrated to obtain the compound of interest (3.18 g, yield: 100%).
NMR (400 MHz, DMSO-d6) δ ppm:2.04 (s, 6H), 2.63 (d, J=5.2 Hz, 3H), 4.17 (brs, 1H), 4.22 (brs, 2H), 4.71 (q, J=5.2 Hz, 1H), 5.93 (dd, J=2.7 Hz, J=8.2 Hz, 1H), 6.03 (d, J=2.7 Hz, 1H), 6.43 (d, J=8.2 Hz, 1H), 6.45 (s, 2H).

### (Example 2)

### N-[(4-Amino-3,5-dimethylphenoxy)-2-(methylamino)phenyl]-2-{4-[(2,4-dioxo-1,3-thiazolidin-5-yl)methyl]phenoxy}acetamide (Compound No: (II)-1, Step 2)

{4-[(2,4-Dioxo-1,3-thiazolidin-5-yl)methyl]phenoxy}acetic acid (3.76 g) was added to a solution of the 4-(4-amino-3,5-dimethylphenoxy)-N²-methylbenzenediamine (3.18 g) obtained in Example 1 in dichloromethane (31 mL), and the mixture was cooled to 0°C. Then, triethylamine (3.73 mL) and subsequently a 50% solution of propylphosphonic acid cyclic anhydride in ethyl acetate (8.69 mL) were added dropwise thereto, and the mixture was stirred at 0°C to 10°C for 40 minutes. A 5% aqueous sodium bicarbonate solution (31 mL) was added thereto, and the mixture was well stirred and then separated into organic and aqueous layers. The organic layer was washed with water (15 mL) and then concentrated to obtain the compound of interest (5.72 g, yield: 97% (based on phenoxyacetic acid)).
NMR (400 MHz, DMSO-d6) δ ppm:2.07 (s, 6H), 2.61 (d, J=4.6 Hz, 3H), 3.08 (dd, J=9.2 Hz, J=14.2 Hz, 1H), 3.31 (dd, J=10.2 Hz, J=14.2 Hz, 1H), 4.63 (s, 2H, 4.89 (dd, J=9.2 Hz, J=10.2 Hz, 1H), 5.17 (brq, J=4.6 Hz, 1H), 5.95 (dd, J=2.6 Hz, J=8.3 Hz, 1H), 6.13 (d, J=2.6 Hz, 1H), 6.56 (s, 2H), 6.89 (d, J=8.3 Hz, 1H), 6.96 (d, J=8.6 Hz, 2H), 7.18 (d, J=8.6 Hz, 2H), 9.11 (s, 1H).

### (Example 3)

### 5-(4{[6-(4-Amino-3,5-dimethylphenoxy)-1-methyl-1H-benzimidazol-2-y]methoxy}benzyl)-1,3-thiazolidine-2,4-dione dihydrochloric acid monohydrate (Step 3)

Concentrated hydrochloric acid (1 mL) was added to a solution of the N-[(4-amino-3,5-dimethylphenoxy)-2-(methylamino)phenyl]-2-{4-[(2,4-dioxo-1,3-thiazolidin-5-yl)methyl]phenoxy}acetamide (1.27 g) obtained in Example 2 in methanol (25 mL) at room temperature, and the mixture was refluxed for approximately 1 hour. The reaction mixture was cooled to room temperature and then concentrated. To the obtained residue, methanol (5 mL) and ethyl acetate (20 mL) were added, and the mixture was stirred for approximately 1 hour. The obtained powder was collected by filtration, washed with a methanol-ethyl acetate (1:4) mixed solvent, and dried under reduced pressure to obtain the compound of interest (1.24 g, yield: 86%).
NMR (400 MHz, DMSO-d6) δ ppm:2.35 (s, 6H), 3.10 (dd, J=8.9 Hz, J=14.1 Hz, 1H), 3.34 (dd, J=4.5 Hz, J=14.1 Hz, 1H), 3.94 (s, 3H), 4.91 (dd, J=4.5 Hz, J=8.9 Hz, 1H), 5.64 (s, 2H), 6.81 (s, 2H), 7.14 (d, J=8.3 Hz, 2H), 7.21 (dd, J=2.1 Hz, J=8.7 Hz, 1H), 7.25 (d, J=8.3 Hz, 2H), 7.64 (d, J=2.1 Hz, 1H), 7.81 (d, J=8.7 Hz, 1H), 12.06 (brs, 1H).

### (Example 4)

### 4-(4-Amino-3,5-dimethylphenoxy)-N²-methylbenzenediamine dihydrochloric acid (dihydrochloride of Compound No: (I)-1, Step 1)

2,6-Di-t-butyl-4-methylphenol (0.13 kg) a 5% palladium carbon catalyst (0.64 kg) were added to a solution of wet crystals of 2,6-dimethyl-4-[3-(methylamino)-4-nitrophenoxy]aniline (13.88 kg, corresponding to a dry weight of approximately 12 kg) in ethyl acetate (126 L). The inside of the system was degassed under reduced pressure, followed by nitrogen substitution. Then, the reaction mixture was heated to approximately 50°C, and the inside of the system was degassed under reduced pressure, followed by hydrogen substitution. The reaction mixture was stirred at the same temperature as above for 3 hours under the hydrogen atmosphere (0.35 MPa), cooled to 20°C, and then filtered. The inside of the reaction container and the residue were washed with a solution of 2,6-di-t-butyl-4-methylphenol (0.09 kg) in 2-propanol (85 L). The filtrate and the washes were combined. 38% hydrochloric acid (21.25 kg) was added dropwise thereto at 20°C, and the mixture was stirred at 5°C for 1 hour and then left standing for 12 hours. The obtained crystals were collected by filtration, washed with ethyl acetate (68 L), and then dried under reduced pressure at 40°C to obtain the compound of interest (12.96 kg, 94%).
NMR (400 MHz, DMSO-d6) δ ppm:2.28 (s, 6H), 2.66 (s, 3H), 6.20 (dd, J=2.3 Hz, J=8.6 Hz, 1H), 6.36 (d, J=2.3 Hz, 1H), 6.74 (s, 2H), 7.17 (d, J=8.6 Hz, 1H), 9.64 (brs, 5H).

### (Example 5)

### 5-(4-{[6-(4-Amino-3,5-dimethylphenoxy)-1-methyl-1H-benzimidazol-2-yl]methoxy}benzyl)-1,3-thiazolidine-2,4-dione dihydrochloric acid monohydrate (Steps 2 and 3)

Tributylamine (7.27 kg) was added to a suspension of {4-[(2,4-dioxo-1,3-thiazolidin-5-yl)methyl]phenoxy}acetic acid (11.04 kg) in tetrahydrofuran (130 L) to prepare a solution. This solution was cooled to -5°C. Pivaloyl chloride (4.73 kg) was added dropwise thereto, and then, the mixture was stirred at -10°C for 30 minutes to obtain a mixed acid anhydride of {4-[(2,4-dioxo-1,3-thiazolidin-5-yl)methyl]phenoxy}acetic acid and pivalic acid. This mixed acid anhydride was added dropwise over 77 minutes to a solution of the 4-(4-amino-3,5-dimethylphenoxy)-N²-methylbenzenediamine dihydrochloric acid (12.96 kg) obtained in Example 4 and tributylamine (24.00 kg) in tetrahydrofuran (130 L) cooled to -10°C, and the mixture was further stirred for 1 hour to obtain a mixed solution of N-[(4-amino-3,5-dimethylphenoxy)-2-(methylamino)phenyl]-2-{4-[(2,4-dioxo-1,3-thiazolidin-5-yl)methyl]phenoxy}acetamide in tetrahydrofuran. The obtained mixed solution was heated to 0°C. Water (130 L) and 38% hydrochloric acid (11.01 kg) were added thereto, and then, the mixture was stirred at 60°C for 30 minutes. The obtained reaction solution was cooled to 5°C and stirred for 12 hours. Then, a 25% aqueous sodium hydroxide solution (19.44 kg) and a solution of sodium chloride (24.92 kg) in water (130 L) were added dropwise thereto at 20°C, and the mixture was stirred for 15 minutes and then left standing for 1.5 hours. The aqueous layer (lower layer) was discarded. A suspension of active carbon (3.89 kg) in tetrahydrofuran (65 L) was added to the obtained solution of 5-(4-{[6-(4-amino-3,5-dimethylphenoxy)-1-methyl-1H-benzimidazol-2-yl]methoxy}benzyl-2,4-dioxo-1,3-thiazolidine (upper layer), and the mixture was stirred at 20°C for 40 minutes. The active carbon was collected by filtration, and the reaction container and the active carbon were washed with tetrahydrofuran (91 L). The filtrate and the washes were combined and stirred at -10°C for 12 hours. This solution was concentrated under reduced pressure at approximately 20°C until its volume became approximately 260 L. To this concentrate, a 38% solution of hydrochloric acid (18.79 kg) in tetrahydrofuran (65 L) was added dropwise at 30°C over 1.5 hours, and the mixture was cooled to 5°C. After further stirring for 1 hour, the obtained crystals were collected by filtration and washed with water (4 L), tetrahydrofuran (35 L), and then water (65 L) to obtain wet crystals of the compound of interest (23.80 kg). A portion of the crystals was sampled and dried under reduced pressure at 40°C, and the NMR spectrum thereof agreed with that obtained in Example 3.

### (Example 6)

### 4-(4-Amino-3,5-dimethylphenoxy)-N²-methylbenzenediamine dihydrochloric acid (dihydrochloride of Compound No: (I)-1, Step 1)

Water (35 mL) and a 50%-wetted 5% palladium carbon catalyst (1.86 g) were added to a solution of 2,6-dimethyl-4-[3-(methylamino)-4-nitrophenoxy]aniline (35.0 g) in tetrahydrofuran (350 mL). The inside of the system was degassed under reduced pressure, followed by nitrogen substitution. The reaction mixture was heated to approximately 60°C, and the inside of the system was further degassed under reduced pressure, followed by hydrogen substitution. The reaction mixture was stirred for 2 hours under the same atmosphere as above (0.1 MPa). The inside of the system was degassed under reduced pressure, followed by nitrogen substitution. Then, the reaction mixture was cooled to 5°C and filtered. The inside of the container and the residue were washed with tetrahydrofuran (70 mL) . The filtrate and the washes were combined and cooled to 0 to 5°C. 38% hydrochloric acid (29.2 g) diluted with tetrahydrofuran (105 mL) was added dropwise thereto over 20 minutes, and the mixture was further stirred at the same temperature as above for 1 hour. The obtained crystals were collected by filtration, washed with tetrahydrofuran (175 mL), and then dried under reduced pressure at 40°C to obtain the compound of interest (38.3 g, yield: 95%). The NMR spectrum of the obtained compound agreed with that obtained in Example 4.

### (Example 7)

### 5-(4-{[6-(4-Amino-3,5-dimethylphenoxy)-1-methyl-1H-benzimidazol-2-yl]methoxy}benzyl)-1,3-thiazolidine-2,4-dione dihydrochloric acid monohydrate (Steps 2 and 3)

Pivaloyl chloride (7.30 g) was added dropwise to a suspension of {4-[(2,4-dioxo-1,3-thiazolidin-5-yl)methyl]phenoxy}acetic acid (17.03 g) in tetrahydrofuran (50 mL) at -20°C, and then, tributylamine (11.22 g) was added dropwise thereto at -20°C to -10°C. The obtained reaction solution was stirred for 1 hour to obtain a mixed acid anhydride of 14-[(2,4-dioxo-1,3-thiazolidin-5-yl)methyl]phenoxy}acetic acid and pivalic acid. This solution of the mixed acid anhydride was added dropwise at -20°C to -10°C over 1 hour to a solution of the 4-(4-amino-3,5-dimethylphenoxy)-N²-methylbenzenediamine dihydrochloric acid (20.00 g) obtained in Example 6 and tributylamine (37.04 g) in tetrahydrofuran (90 mL) cooled to -20°C, and the reaction container of the mixed acid anhydride was further washed with tetrahydrofuran (30 mL). The reaction solution was stirred at the same temperature as above for 1 hour to obtain a mixed solution of N-[(4-amino-3,5-dimethylphenoxy)-2-(methylamino)phenyl]-2-{4-[(2,4-dioxo-1,3-thiazolidin-5-yl)methyl]phenoxy}acetamide in tetrahydrofuran. Water (64 mL) and 38% hydrochloric acid (12.20 g) were added thereto, and then, the mixture was refluxed for 3 hours to obtain a mixed solution of 5-(4-{[6-(4-amino-3,5-dimethylphenoxy)-1-methyl-1H-benzimidazol-2-yl]methoxy}benzyl-2,4-dioxo-1,3-thiazolidine and hydrochloride. To the obtained reaction solution, tributylamine (8.98 g) and tetrahydrofuran (240 mL) were added dropwise at 60°C to prepare a solution of 5-(4-{[6-(4-amino-3,5-dimethylphenoxy)-1-methyl-1H-benzimidazol-2-yl]methoxy}benzyl-2,4-dioxo-1,3-thiazolidine in water and tetrahydrofuran. A suspension of active carbon (3.4 g) in tetrahydrofuran (30 mL) degassed under reduced pressure was added thereto. This mixture was stirred at 60°C for 1 hour. Then, the active carbon was collected by filtration, and the inside of the reaction container and the active carbon were washed with tetrahydrofuran (70 mL). The filtrate and the washes were combined and heated to 50°C. Then, water (86 mL) and subsequently 38% hydrochloric acid (29.05 g) were added dropwise thereto, and the mixture was stirred at the same temperature as above for 12 hours. The obtained crystals were collected by filtration, washed with tetrahydrofuran (120 mL), and then dried at 3.3 kPa at 50°C to obtain the compound of interest (32.93 g, 92% (based on phenoxyacetic acid)). The NMR spectrum of this compound agreed with that obtained in Example 3.

### (Example 8)

### t-Butyl {4-[4-amino-3-(methylamino)phenoxy]-2,6-dimethylphenyl}carbamate (Compound No: (I)-10, Step 1)

A 50%-wetted 10% palladium carbon catalyst (200 mg) was added to a mixed solution of t-butyl {2,6-dimethyl-4-[3-(methylamino)-4-nitrophenoxy]phenylcarbamate (2.0 g) in methanol (23 mL) and tetrahydrofuran (5 mL) at 40°C. The inside of the system was degassed, followed by hydrogen substitution. The mixture was stirred at 40°C for 3 hours under the hydrogen atmosphere (3 atmospheric pressures). The reaction mixture was cooled to room temperature. Then, the palladium carbon was collected by filtration, and the inside of the reaction container and the palladium carbon were washed with methanol (40 mL). The filtrate and the washes were combined and concentrated under reduced pressure to obtain the compound of interest (1.94 g, yield: 100%). HPLC (column: L-column ODS 4.6 x 250 mm, mobile phase: acetonitrile-0.02 M aqueous ammonium acetate solution = 6:4, flow rate: 1.0 mL/min, column temperature: 40°C, detection wavelength: 220 nm) retention time (min): 7.34.

### (Example 9)

### t-Butyl (4-{4-[({4-[(2,4-dioxo-1,3-thiazolidin-5-yl)methyl]phenoxy}acetyl)amino]-3-(methylamino)phenoxy}-2,6-dimethylphenyl)carbamate (Compound No: (II)-10, Step 2)

Pivaloyl chloride (320 mg) and subsequently tributylamine (493 mg) were added dropwise to a solution of {4-[(2,4-dioxo-1,3-thiazolidin-5-yl)methyl]phenoxy}acetic acid (747 mg) in ethyl acetate (6 mL) at 0°C, and the mixture was stirred for 30 minutes. The obtained solution of the mixed acid anhydride was added dropwise to a solution of the t-butyl {4-[4-amino-3-(methylamino)phenoxy]-2,6-dimethylphenyl}carbamate (970 mg) obtained in Example 8 and triethylamine (717 mg) in ethyl acetate (8 mL) at 0°C, and the mixture was stirred at the same temperature as above for 1 hour. Ethyl acetate (10 mL) and water (20 mL) were poured to the reaction mixture, and the organic layer was separated, then washed with 10% hydrochloric acid (20 mL) twice, a 5% aqueous sodium bicarbonate solution (20 mL) twice, and water (20 mL) once, and dried over anhydrous magnesium sulfate. The anhydrous magnesium sulfate was filtered off, and then, the organic layer was concentrated to obtain the compound of interest.
HPLC (column: L-column ODS 4.6 x 250 mm, mobile phase: acetonitrile-0.02 M aqueous ammonium acetate solution = 1:1, flow rate: 1.0 mL/min, column temperature: 40°C, detection wavelength: 220 nm) retention time (min): 12.01.

### (Example 10)

### 5-(4-{[6-(4-Amino-3,5-dimethylphenoxy)-1-methyl-1H-benzimidazol-2-yl]methoxy}benzyl)-1,3-thiazolidine-2,4-dione dihydrochloric acid monohydrate (Step 3)

38% hydrochloric acid (1238 mg) was added to a solution of the t-butyl (4-{4-({4-[(2,4-dioxo-1,3-thiazolidin-5-yl)methyl]phenoxy}acetyl)amino]-3-(methylamino)phenoxy-2,6-dimethylphenyl}carbamate obtained in Example 9 in methanol (20 mL) at room temperature, and the mixture was stirred for 1 hour, further refluxed for 1 hour, and then cooled to 0°C. The obtained crystals were collected by filtration, washed with methanol (10 mL), and then dried under reduced pressure to obtain the compound of interest (1090 mg, yield: 69% (based on phenoxyacetic acid)). The NMR spectrum of this compound agreed with that obtained in Example 3.

### (Example 11)

### 5-(4-{[6-(4-Amino-3,5-dimethylphenoxy)-1-methyl-1H-benzimidazol-2-yl]methoxy}benzyl)-1,3-thiazolidine-2,4-dione dihydrochloric acid monohydrate

A suspension of 5-(4-{[6-(4-amino-3,5-dimethylphenoxy)-1-methyl-1H-benzimidazol-2-yl]methoxy}benzyl)-1,3-thiazolidine-2,4-dione dihydrochloric acid monohydrate (22.50 kg) obtained according to the method of Example 7 in water (450 L) was stirred at 80°C for 30 minutes. Then, hydrochloric acid (38% hydrochloric acid (14.55 kg) diluted with water (113 L)) was added thereto over 1.5 hours. The mixture was stirred for 1 hour, then cooled to 40°C, and stirred for 18 hours. The obtained crystals were collected by filtration, washed with hydrochloric acid (38% hydrochloric acid (1.75 kg) diluted with water (68 L)), and then dried at 4.3 kPa at 50°C to obtain the compound of interest (21.49 kg, yield: 96%). The diffraction pattern of this compound in powder X-ray diffraction (CuKα, λ = 1.54 angstroms) is shown in Figure 1. When the maximum peak intensity in the diffraction pattern shown in Figure 1 is defined as 100, peaks having a relative intensity of 10 or more are shown in Table 2. Peak Nos. in Figure 1 correspond to Peak Nos. in Table 2.

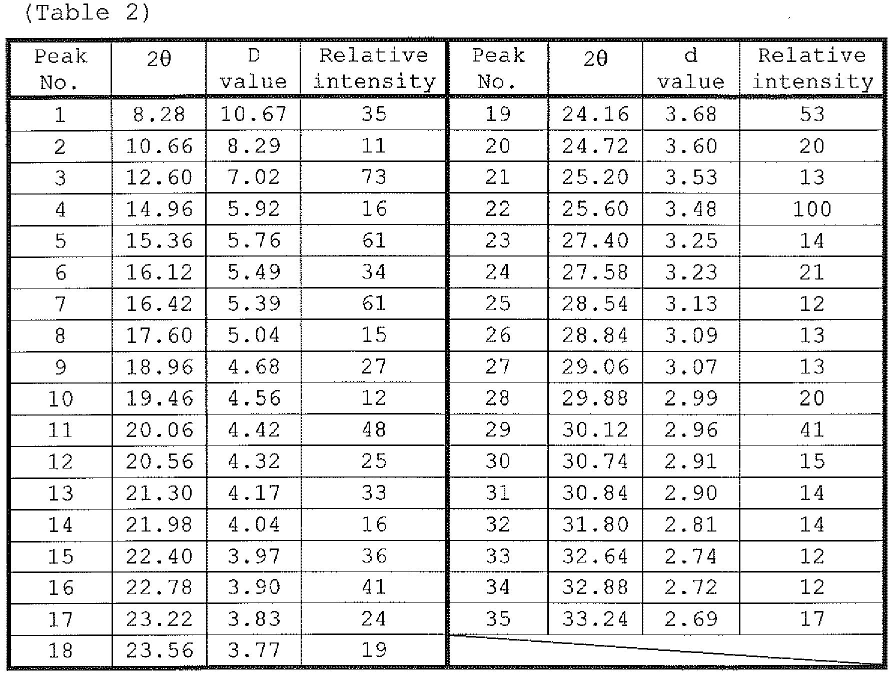

### (Test Example 1)

Purity measurement of 5-(4-{[6-(4-amino-3,5-dimethylphenoxy)-1-methyl-1H-benzimidazol-2-yl]methoxy}benzyl)-1,3-thiazolidine-2,4-dione dihydrochloric acid monohydrate

The title compound obtained in Example 7 was analyzed under the following HPLC conditions, and results of measuring its purity are shown in Table 2. Column: XTerra RP18 (manufactured by Waters Corp., 4.6 mm x 150 mm)
Column temperature: constant temperature of approximately 40°C
Mobile phase: 0.01 M ammonium acetate buffer (pH 4.5): acetonitrile
= 65:35
Flow rate: approximately 1 mL/min. (which was set so that the retention time of the compound of interest was approximately 25 min.)

### Detection wavelength: 230 nm

Sample solution preparation method: approximately 0.05 g of the analyte compound is weighed into a 50-mL volumetric flask and dissolved by the addition of a water-acetonitrile (3:2) mixed solution, and then, a water-acetonitrile (3:2) mixed solution is further added thereto to bring the volume of the solution to 50 mL. Measurement time: 70 minutes after injection of the sample solution

**(Table 3)**

| Peak No. | Retention time (min) | Relative area ratio (%) | Remarks |
|---|---|---|---|
| 1 | 2.38 | 0.003 | |
| 2 | 3.21 | 0.008 | |
| 3 | 3.37 | 0.016 | |
| 4 | 4.00 | 0.009 | |
| 5 | 4.25 | 0.005 | |
| 6 | 4.51 | 0.002 | |
| 7 | 4. 93 | 0.046 | |
| 8 | 5.13 | 0.018 | |
| 9 | 5.57 | 0.256 | |
| 10 | 6. 98 | 0.004 | |
| 11 | 7.84 | 0.164 | |
| 12 | 9.02 | 0.018 | |
| 13 | 10.61 | 0.028 | |
| 14 | 11.52 | 0.133 | |
| 15 | 15.68 | 0.009 | |
| 16 | 16.15 | 0.011 | |
| 17 | 25.12 | 98.76 | Compound of interest |
| 18 | 47.80 | 0.012 | |
| 19 | 52.74 | 0.012 | |
| 20 | 54.32 | 0.010 | |
| 21 | 59.56 | 0.480 | |

## Claims

1. A method for manufacturing a 6-substituted 1-methyl-1H-benzimidazole derivative represented by the following general formula (IV) or a salt thereof, or a hydrate thereof, comprising subjecting a compound represented by the following general formula (II) to intramolecular dehydration condensation in the presence of an acid, and, if necessary, deprotecting a protective group for the nitrogen atom: 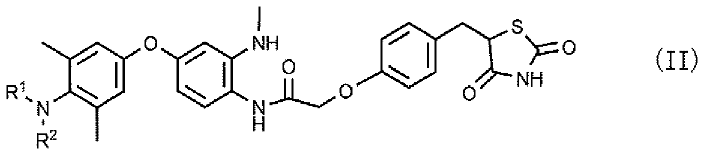 wherein R¹ represents a hydrogen atom, a C₁-C₄ alkyl group, or a protective group for the nitrogen atom; and R² represents a hydrogen atom or a protective group for the nitrogen atom, and 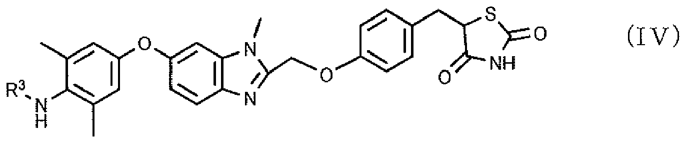 wherein R³ represents a hydrogen atom or a C₁-C₄ alkyl group.

2. The manufacturing method according to claim 1, wherein the acid is hydrochloric acid, R¹ represents a hydrogen atom, and R² represents a hydrogen atom or a t-butoxycarbonyl group.

3. A compound represented by the following general formula (II): 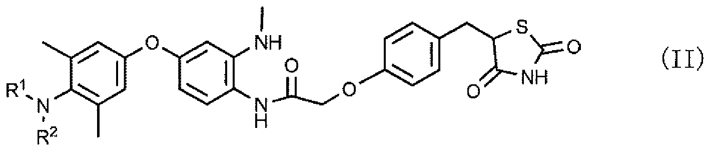 wherein R¹ represents a hydrogen atom, a C₁-C₄ alkyl group, or a protective group for the nitrogen atom; and R² represents a hydrogen atom or a protective group for the nitrogen atom.

4. The compound according to claim 3, wherein R¹ represents a hydrogen atom and R² represents a hydrogen atom or a t-butoxycarbonyl group.

5. A method for manufacturing a compound represented by the following general formula (II), comprising reacting a 4-substituted N²-methylbenzene-1,2-diamine derivative represented by the following general formula (I) with {4-[(2,4-dioxo-1,3-thiazolidin-5-yl)methyl]phenoxy}acetic acid in the presence of a condensing agent: 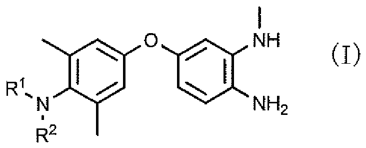 wherein R¹ represents a hydrogen atom, a C₁-C₄ alkyl group, or a protective group for the nitrogen atom; and R² represents a hydrogen atom or a protective group for the nitrogen atom, and 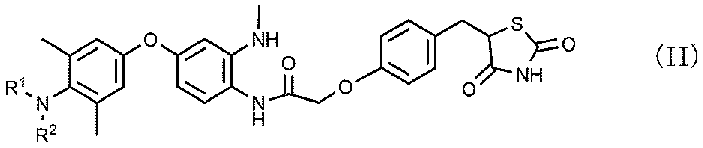 wherein R¹ represents a hydrogen atom, a C₁-C₄ alkyl group, or a protective group for the nitrogen atom; and R² represents a hydrogen atom or a protective group for the nitrogen atom.

6. The manufacturing method according to claim 5, wherein R¹ represents a hydrogen atom and R² represents a hydrogen atom or a t-butoxycarbonyl group.

7. A method for manufacturing a compound represented by the following general formula (II), comprising reacting a 4-substituted N²-methylbenzene-1,2-diamine derivative represented by the following general formula (I) with an acid chloride or a mixed acid anhydride of {4-[(2,4-dioxo-1,3-thiazolidin-5-yl)methyl]phenoxy}acetic acid: 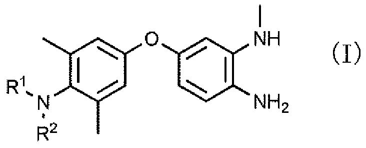 wherein R¹ represents a hydrogen atom, a C₁-C₄ alkyl group, or a protective group for the nitrogen atom; and R² represents a hydrogen atom or a protective group for the nitrogen atom, and 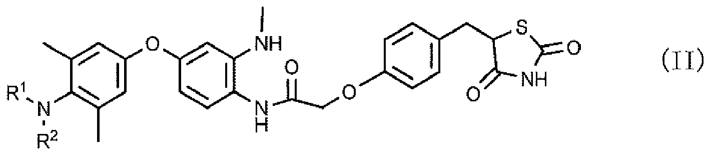 wherein R¹ represents a hydrogen atom, a C₁-C₄ alkyl group, or a protective group for the nitrogen atom; and R² represents a hydrogen atom or a protective group for the nitrogen atom.

8. The manufacturing method according to claim 7, wherein R¹ represents a hydrogen atom, and R² represents a hydrogen atom or a t-butoxycarbonyl group.

9. A 4-substituted N²-methylbenzene-1,2-diamine derivative represented by the following general formula (I) or a salt thereof: 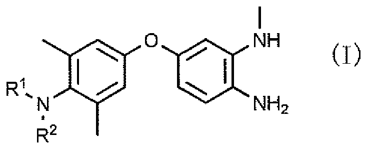 wherein R¹ represents a hydrogen atom, a C₁-C₄ alkyl group, or a protective group for the nitrogen atom; and R² represents a hydrogen atom or a protective group for the nitrogen atom.

10. The 4-substituted N²-methylbenzene-1,2-diamine derivative according to claim 9 or a salt thereof, wherein R¹ represents a hydrogen atom, and R² represents a hydrogen atom or a t-butoxycarbonyl group.

11. A method for manufacturing a 4-substituted N²-methylbenzene-1,2-diamine derivative represented by the following general formula (I), comprising reducing a 5-substituted N-methyl-2-nitroaniline derivative represented by the following general formula (III) by hydrogenation: 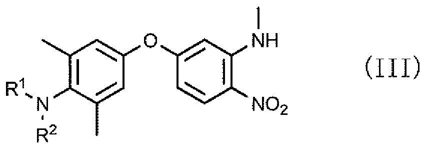 wherein R¹ represents a hydrogen atom, a C₁-C₄ alkyl group, or a protective group for the nitrogen atom; and R² represents a hydrogen atom or a protective group for the nitrogen atom, and 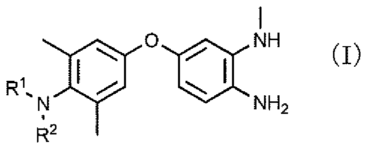 wherein R¹ represents a hydrogen atom, a C₁-C₄ alkyl group, or a protective group for the nitrogen atom; and R² represents a hydrogen atom or a protective group for the nitrogen atom.

12. The manufacturing method according to claim 11, wherein R¹ represents a hydrogen atom, and R² represents a hydrogen atom or a t-butoxycarbonyl group.

13. A method for manufacturing a 6-substituted 1-methyl-1H-benzimidazole derivative represented by the following general formula (IV) or a salt thereof, or a hydrate thereof, comprising
reducing a 5-substituted N-methyl-2-nitroaniline derivative represented by the following general formula (III) by hydrogenation to produce a 4-substituted N²-methylbenzene-1,2-diamine derivative represented by the following general formula (I),
subsequently reacting the 4-substituted N²-methylbenzene-1,2-diamine derivative represented by the formula (I) with {4-[(2,4-dioxo-1,3-thiazolidin-5-yl)methyl]phenoxy}acetic acid in the presence of a condensing agent or reacting the 4-substituted N²-methylbenzene-1,2-diamine derivative represented by the formula (I) with an acid chloride or a mixed acid anhydride of {4-[(2,4-dioxo-1,3-thiazolidin-5-yl)methyl]phenoxy}acetic acid to produce a compound represented by the following general formula (II),
subsequently subjecting the compound represented by the formula (II) to intramolecular dehydration condensation in the presence of an acid, and, if necessary, deprotecting a protective group for the nitrogen atom: 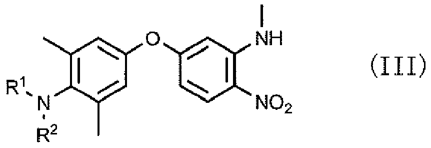 wherein R¹ represents a hydrogen atom, a C₁-C₄ alkyl group, or a protective group for the nitrogen atom; and R² represents a hydrogen atom or a protective group for the nitrogen atom, 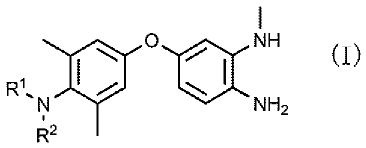 wherein R¹ represents a hydrogen atom, a C₁-C₄ alkyl group, or a protective group for the nitrogen atom; and R² represents a hydrogen atom or a protective group for the nitrogen atom, 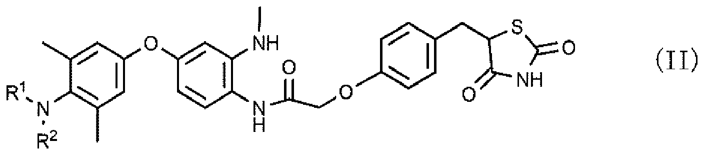 wherein R¹ represents a hydrogen atom, a C₁-C₄ alkyl group, or a protective group for the nitrogen atom; and R² represents a hydrogen atom or a protective group for the nitrogen atom, and 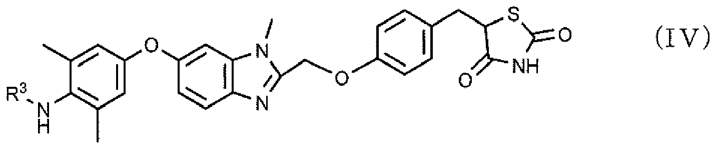 wherein R³ represents a hydrogen atom or a C₁-C₄ alkyl group.
